# EUROPEAN PATENT APPLICATION

(11) **EP 4 786 470 A1**
(43) Date of publication of application: **05.08.2026**
(21) Application number: 24872696.0
(22) Date of filing: 29.07.2024
(51) Int. Cl.: C07D 451/02, C07B 63/00, C07B 59/00, A61K 51/04

(54) **METHOD FOR STABILIZING FP-CIT LINEAR PRECURSOR WITH HIGH PURITY, AND METHOD FOR PREPARING [18F]FP-CIT BY USING SAME**

(30) Priority: 26.09.2023 KR 20230129820; 18.03.2024 KR 20240037443
(71) Applicant: The Asan Foundation, Seoul 05505 (KR)
(72) Inventor: KO, Nare, Seoul 05505 (KR)
(74) Representative: Dehns
(86) International application number: PCT/KR2024/010995
(87) International publication number: WO 2025/070987

(57) **Abstract**

The present invention relates to a method for stabilizing (storing and pre-treating) an FP-CIT linear precursor with a high purity, and a method for preparing [¹⁸F]FP-CIT by using same. If an organic solvent having characteristics according to the present invention is used for storing an FP-CIT linear precursor, conversion of the FP-CIT linear precursor into a cyclic salt form or decomposition of the FP-CIT linear precursor into various compounds is suppressed to enable high purity of the FP-CIT linear precursor to be maintained, and [¹⁸F]FP-CIT, which is a radiopharmaceutical, can be prepared in high yield by using a conventional [¹⁸F]FP-CIT labeling condition as is, and thus the present invention can be effectively used as a starting material of [¹⁸F]FP-CIT.

## Description

### [Technical Field]

The present invention relates to a method for stabilizing (storing, pre-treating) an FP-CIT linear precursor at a high purity and a method for preparing [¹⁸F]FP-CIT using the same.

This application claims priority to and the benefit of Korean Patent Application No. 10-2023-0129820, filed on September 26, 2024, and Korean Patent Application No. 10-2024-0037443, filed on March 18, 2024, the disclosure of which is incorporated herein by reference in its entirety.

### [Background Art]

[¹⁸F] fluoride-labeled radiopharmaceuticals are diagnostic radiopharmaceuticals, and have the characteristic of being prepared and used on the day of use due to the short half-life of [¹⁸F] fluoride, which is 110 minutes. The preparation step of an [¹⁸F] fluoride-labeled radiopharmaceutical involves the steps of producing and activating [¹⁸F] fluoride, labeling a radiopharmaceutical precursor with [¹⁸F] fluoride, and purifying and formulating the [¹⁸F] fluoride-labeled radiopharmaceutical. In this case, the radiopharmaceutical precursor typically has a leaving group at a position where [¹⁸F] fluoride is labeled, and [¹⁸F] fluoride is labeled through a nucleophilic substitution reaction.

As a precursor of [¹⁸F]FP-CIT, N-(3'-methansulfonyloxipropyl)-2-β-carbomethoxy-3-β-(4'-iodophenyl)tropane or N-(3'-toluenesulfonyloxipropyl)-2-β-carbomethoxy-3-β-(4'-iodophenyl)tropane is used as an FP-CIT linear precursor, the corresponding compound is in a form in which a propyl having a leaving group at its end is present in a tertiary amine, and in this case, it has been reported that a cyclic salt with a square ring is formed through an intramolecular cyclization reaction.

As described above, the mixture of linear precursor structures and cyclic salt precursors due to a phenomenon in which the compound is spontaneously converted into a cyclic salt occurring due to the structural characteristics of the compounds not only affects the purity of the starting materials of a radiopharmaceutical, but also affects the preparation yield of the radiopharmaceutical.

Therefore, as a result of conducting extensive research to find a method capable of stably maintaining a linear structure by suppressing the natural change of an FP-CIT linear precursor, that is, the conversion of the linear precursor into a cyclic salt, the present inventors have not only established a stabilization method capable of securing the long-term storage stability of the FP-CIT linear precursor, but also used the method to prepare an [¹⁸F]FP-CIT with high labeling efficiency, thereby completing the present invention.

### [Disclosure]

### [Technical Problem]

As a result of conducting extensive research to find a method capable of stably maintaining a linear structure by suppressing the conversion of an FP-CIT linear precursor used for the preparation of [¹⁸F]FP-CIT into a cyclic salt precursor, the present inventors have not only established a stabilization method capable of securing the long-term storage stability of the FP-CIT linear precursor, but also used the method to prepare an [¹⁸F]FP-CIT with high labeling efficiency, thereby completing the present invention.

The present invention is directed to providing a liquid composition for storing an FP-CIT linear precursor, including an organic solvent having the following characteristics:
(a) a boiling point of 10 to 100°C;
(b) an evaporation degree of 50 to 400 Torr; and
(c) a water solubility of 0.1 to 50%.

The present invention is also directed to providing a composition for improving the storage stability of an FP-CIT linear precursor, including an organic solvent having the following characteristics:
(a) a boiling point of 10 to 100°C;
(b) an evaporation degree of 50 to 400 Torr; and
(c) a water solubility of 0.1 to 50%.

The present invention is also directed to providing a method for stabilizing an FP-CIT linear precursor by storing the FP-CIT linear precursor in the composition according to the present invention.

The present invention is also directed to providing a method for preparing an [¹⁸F]FP-CIT using the composition according to the present invention including an FP-CIT linear precursor as a starting material.

The present invention is also directed to providing a method for preparing [¹⁸F]FP-CIT, the method including an FP-CIT linear precursor pretreatment step of dissolving an FP-CIT linear precursor in an organic solvent having the following characteristics:
(a) a boiling point of 10 to 100°C;
(b) an evaporation degree of 50 to 400 Torr; and
(c) a water solubility of 0.1 to 50%.

The present invention is also directed to providing a kit for storing an FP-CIT linear precursor.

The present invention is also directed to providing a kit for preparing [¹⁸F]FP-CIT.

However, the technical problems which the present invention intends to solve are not limited to the technical problems that have been mentioned above, and other technical problems that have not been mentioned will be clearly understood by a person with ordinary skill in the art to which the present invention pertains from the following description.

### [Technical Solution]

According to an aspect of the present invention, there is provided a liquid composition for storing an FP-CIT linear precursor, including an organic solvent having the following characteristics:
(a) a boiling point of 10 to 100°C;
(b) an evaporation degree of 50 to 400 Torr; and
(c) a water solubility of 0.1 to 50%.

According to another aspect of the present invention, there is provided a composition for improving the storage stability of an FP-CIT linear precursor, including an organic solvent having the following characteristics:
(a) a boiling point of 10 to 100°C;
(b) an evaporation degree of 50 to 400 Torr; and
(c) a water solubility of 0.1 to 50%.

According to still another aspect of the present invention, there is provided a method for stabilizing an FP-CIT linear precursor by storing an FP-CIT linear precursor in the composition according to the present invention.

As an exemplary embodiment of the present invention, the organic solvent may be one or more selected from the group consisting of tetrahydrofuran (THF), ethyl acetate (EA), chloroform (CHCl₃), and dichloromethane (DCM), but is not limited thereto.

As another exemplary embodiment of the present invention, the FP-CIT linear precursor may be a compound represented by the following Chemical Formula 1, but is not limited thereto: 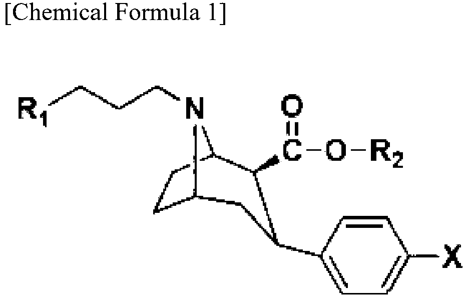 (in Chemical Formula 1,
R₁ is sulfonyl or a halogen,
R₂ is a C₁₋₅ alkyl, and
X is a halogen).

As still another exemplary embodiment of the present invention, R₁ is methanesulfonyl (mesyl), toluenesulfonyl (tosyl), nitrobenzenesulfonyl (nosyl), F, Cl, Br, or I,
R₂ is methyl or ethyl, and
X may be Cl, Br, or I, but is not limited thereto.

As yet another exemplary embodiment of the present invention, the FP-CIT linear precursor may be N-(3'-methansulfonyloxipropyl)-2-β-carbomethoxy-3-β-(4'-iodophenyl)tropane or N-(3'-toluenesulfonyloxipropyl)-2-β-carbomethoxy-3-β-(4'-iodophenyl)tropane, but is not limited thereto.

As yet another exemplary embodiment of the present invention, the storage temperature of the FP-CIT linear precursor may be -50 to 0°C, but is not limited thereto.

As yet another exemplary embodiment of the present invention, the FP-CIT linear precursor may be stored at a concentration of 0.1 to 16 mg/mL, but the concentration is not limited thereto.

As yet another exemplary embodiment of the present invention, the liquid composition may suppress the conversion of the FP-CIT linear precursor into an FP-CIT cyclic salt precursor, but is not limited thereto.

As yet another exemplary embodiment of the present invention, the FP-CIT linear precursor may maintain 50 to 99% of the initially stored amount of FP-CIT linear precursor when stored for 12 months, but is not limited thereto.

As yet another exemplary embodiment of the present invention, when the FP-CIT linear precursor is stored for 12 months, 1% to 50% of the initially stored amount of FP-CIT linear precursor may be converted into an FP-CIT cyclic salt precursor, but is not limited thereto.

As yet another exemplary embodiment of the present invention, the FP-CIT linear precursor may have a ratio (FP-CIT cyclic salt precursor)/(FP-CIT linear precursor) of 0.01 to 1 when stored for 12 months, but is not limited thereto.

As yet another exemplary embodiment of the present invention, the improvement in storage stability of the FP-CIT linear precursor may suppress the conversion of the FP-CIT linear precursor into an FP-CIT cyclic salt precursor, but is not limited thereto.

According to yet another aspect of the present invention, there is provided a method for preparing [¹⁸F]FP-CIT using the composition according to the present invention including an FP-CIT linear precursor as a starting material, the method including:
(a) a step of isolating the FP-CIT linear precursor by removing an organic solvent from the composition according to the present invention including the FP-CIT linear precursor;
(b) a pre-labeling reaction step of preparing a mixture by dissolving the FP-CIT linear precursor isolated in Step (a) in a reaction solvent; and
(c) a labeling reaction step of labeling (with [¹⁸F]fluoride) by adding the mixture in Step (b) to a reaction vessel including activated [¹⁸F]fluoride.

According to yet another aspect of the present invention, there is provided a method for preparing [¹⁸F]FP-CIT using the composition according to the present invention including an FP-CIT linear precursor as a starting material, the method including:
(a) a step of preparing a mixture by adding the composition according to the present invention including an FP-CIT linear precursor to a reaction vessel including activated [¹⁸F]fluoride;
(b) a pre-labeling reaction step of removing an organic solvent from the mixture in Step (a); and
(c) a labeling reaction step of labeling (with [¹⁸F]fluoride) by adding a reaction solvent to the mixture from which the organic solvent has been removed.

As an exemplary embodiment of the present invention, the [¹⁸F]FP-CIT may be prepared through a nucleophilic fluorination reaction of an FP-CIT linear precursor with activated [¹⁸F] fluoride, but is not limited thereto.

As another exemplary embodiment of the present invention, the reaction solvent may be a polar organic solvent, but is not limited thereto.

As still another exemplary embodiment of the present invention, the polar organic solvent may be one or more protic tertiary alcohols selected from the group consisting of t-butanol, t-amyl alcohol, and 1-methoxy-2-methyl-2-propanol, but is not limited thereto.

As yet another exemplary embodiment of the present invention, the polar organic solvent may be one or more aprotic organic solvents selected from the group consisting of acetonitrile (CH₃CN), dimethyl sulfoxide (DMSO), and dimethylformamide (DMF), but is not limited thereto.

As yet another exemplary embodiment of the present invention, the activated [¹⁸F]fluoride may be activated with tetra-n-butylammonium or 4,7,13,16,21,24-hexaoxa-1,10-diazabicyclo[8.8.8]hexacosane (Kryptofix^{™} 222), but is not limited thereto.

According to yet another aspect of the present invention, there is provided a method for preparing [¹⁸F]FP-CIT, the method including: an FP-CIT linear precursor pretreatment step of dissolving an FP-CIT linear precursor in an organic solvent having the following characteristics:
(a) a boiling point of 10 to 100°C;
(b) an evaporation degree of 50 to 400 Torr; and
(c) a water solubility of 0.1 to 50%.

As an exemplary embodiment of the present invention, the method may further include the following steps, but is not limited thereto:
(a) a step of isolating an FP-CIT linear precursor by removing an organic solvent from the organic solvent in which the FP-CIT linear precursor is dissolved;
(b) a labeling reaction pre-step of preparing a mixture by dissolving the FP-CIT linear precursor isolated in Step (a) in a reaction solvent; and
(c) a labeling reaction step of labeling (with [¹⁸F]fluoride) by adding the mixture in Step (b) to a reaction vessel including activated [¹⁸F]fluoride.

As another exemplary embodiment of the present invention, the method may further include the following steps, but is not limited thereto:
(a) preparing a mixture by adding an organic solvent in which an FP-CIT linear precursor is dissolved to a reaction vessel including activated [¹⁸F]fluoride;
(b) pre-labeling reaction step of removing an organic solvent from the mixture in Step (a); and
(c) labeling reaction step of the labeling (with [¹⁸F]fluoride) by adding a reaction solvent to the mixture from which the organic solvent has been removed.

According to yet another aspect of the present invention, there is provided a kit for storing an FP-CIT linear precursor, including the composition according to the present invention.

According to yet another aspect of the present invention, there is provided a kit for preparing [¹⁸F]FP-CIT, including a first kit, a second kit, and instructions,
wherein the first kit includes a liquid composition for storing the FP-CIT linear precursor according to the present invention, including an FP-CIT linear precursor, and
the second kit includes activated [¹⁸F]fluoride and a reaction solvent.

### [Advantageous Effects]

When the organic solvent having the characteristics according to the present invention is used for storing (preserving) an FP-CIT linear precursor, the organic solvent can be usefully used for the preparation of [¹⁸F]FP-CIT because it is possible not only to maintain the FP-CIT linear precursor at high purity by suppressing the conversion of the FP-CIT linear precursor into a cyclic salt form or degradation into various compounds, but also to synthesize a radiopharmaceutical [¹⁸F]FP-CIT at a high yield using the existing [¹⁸F]FP-CIT labeling conditions as they are.

### [Description of Drawings]

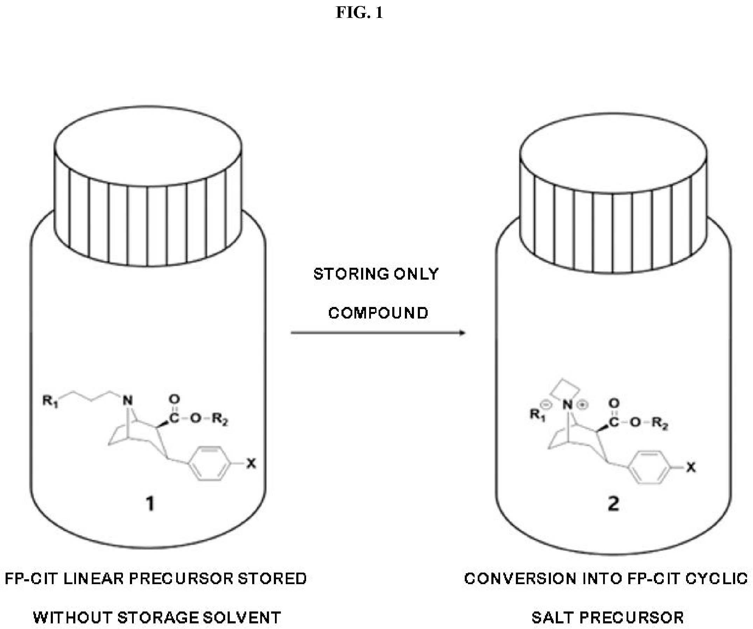

FIG. 1 is a view illustrating a method for storing a conventional FP-CIT linear precursor.
FIG. 2 is a view illustrating a method for storing an FP-CIT linear precursor according to the present invention.
FIG. 3 is a view illustrating the results of confirming the purity of an FP-CIT linear precursor stored at a storage concentration of 1 mg/mL at room temperature, according to the type of storage solvent.
FIG. 4 is a view illustrating the results of confirming the purity of an FP-CIT linear precursor stored at a storage concentration of 1 mg/mL at -20°C, according to the type of storage solvent.
FIG. 5 is a view illustrating the results of confirming the purity of an FP-CIT linear precursor stored in tetrahydrofuran (THF) or ethyl acetate (EA) at a storage concentration of 8 mg/mL at room temperature or -20°C.
FIG. 6 is a view illustrating a method for synthesizing [¹⁸F]FP-CIT using the FP-CIT linear precursor stored according to the present invention.
FIG. 7 is a view illustrating a method for synthesizing [¹⁸F]FP-CIT using the FP-CIT linear precursor stored according to the present invention.

### [Best Mode]

Due to its structural characteristics, an FP-CIT linear precursor, which is a starting material for the preparation of a radiopharmaceutical [¹⁸F]FP-CIT, begins to be converted into a cyclic salt precursor from the time point when the synthesis is completed, and the ratio of the precursor gradually decreases, which has been identified as a problem that cannot be solved even by storing the precursor in a solid state at a low temperature.

Therefore, although a method of preparing a linear salt precursor using a tertiary amine with a propyl having a leaving group and a specific acid (for example, methanesulfonic acid, p-toluenesulfonic acid, and the like) was developed in order to solve these problems, which improved the linearity of the precursor, another problem occurred in which the [¹⁸F]fluoride labeling conditions changed due to the acid that formed a salt, leading to the failure to prepare [¹⁸F]FP-CIT, and therefore there is a need to develop a method for storing the FP-CIT linear precursor for a long period of time.

Therefore, as a result of conducting extensive research to find a method capable of suppressing the conversion of an FP-CIT linear precursor into a cyclic salt precursor and stably maintaining a linear structure, the present inventors not only established a method for stabilizing the FP-CIT linear precursor by confirming that when the FP-CIT linear precursor is stored in an organic solvent under specific conditions at a specific temperature, the FP-CIT linear precursor can be stably stored (preserved) for a long period of time, but also confirmed that [¹⁸F]FP-CIT prepared using the method exhibits high [¹⁸F]fluoride labeling efficiency, thereby completing the present invention.

Hereinafter, the present invention will be described in detail.

The present invention provides a liquid composition for storing an FP-CIT linear precursor or a composition for improving the storage stability of an FP-CIT linear precursor, including an organic solvent having the following characteristics:
(a) a boiling point of 10 to 100°C;
(b) an evaporation degree of 50 to 400 Torr; and
(c) a water solubility of 0.1 to 50%.

In addition, the present invention provides a liquid composition for storing an FP-CIT linear precursor or a composition for improving the storage stability of an FP-CIT linear precursor, consisting of an organic solvent having the following characteristics:
(a) a boiling point of 10 to 100°C;
(b) an evaporation degree of 50 to 400 Torr; and
(c) a water solubility of 0.1 to 50%.

In addition, the present invention provides a method for stabilizing an FP-CIT linear precursor by storing an FP-CIT linear precursor in the composition according to the present invention.

In the present invention, the boiling point may be 10 to 100°C, 10 to 90°C, 10 to 80°C, 10 to 70°C, 10 to 60°C, 10 to 50°C, 10 to 40°C, 10 to 30°C, 30 to 100°C, 30 to 90°C, 30 to 80°C, 30 to 70°C, 30 to 60°C, 60 to 100°C, 60 to 90°C, 60 to 80°C, 60 to 78°C, 63 to 78°C, 65 to 78°C, 66 to 78°C, 66 to 77.5°C, 66 to 77°C, 68 to 78°C, 70 to 78°C, 73 to 78°C, 75 to 78°C, 65 to 75°C, 65 to 73°C, 65 to 70°C, 65 to 67°C, 66°C, 67°C, 68°C, 69°C, 70°C, 71°C, 72°C, 73°C, 74°C, 75°C, 76°C, 77°C, or 77.1°C, but is not limited thereto.

In the present invention, the evaporation degree (vapor pressure) measured at 20°C may be 50 to 400 Torr(mmHg), 50 to 380 Torr, 50 to 350 Torr, 50 to 330 Torr, 50 to 300 Torr, 50 to 200 Torr, 50 to 180 Torr, 50 to 160 Torr, 50 to 150 Torr, 60 to 350 Torr, 60 to 300 Torr, 60 to 200 Torr, 60 to 180 Torr, 60 to 160 Torr, 60 to 150 Torr, 70 to 200 Torr, 70 to 180 Torr, 70 to 160 Torr, 70 to 150 Torr, 70 to 145 Torr, 70 to 144 Torr, 70 to 143 Torr, 70 to 142 Torr, 70 to 141 Torr, 70 to 140 Torr, 71 to 144 Torr, 71 to 143 Torr, 71 to 142 Torr, 71 to 141 Torr, 72 to 143 Torr, 72 to 142 Torr, 73 to 142 Torr, 73 Torr, or 142 Torr, but is not limited thereto.

In the present invention, the water solubility at 20°C may be 0.1 to 50%, 0.1 to 40%, 0.1 to 30%, 0.5 to 50%, 0.5 to 40%, 0.5 to 30%, 0.8 to 50%, 0.8 to 40%, 0.8 to 30%, 1 to 50%, 1 to 40%, 1 to 30%, 1.5 to 50%, 1.5 to 40%, 1.5 to 30%, 2 to 30%, 3 to 30%, 5 to 30%, 8 to 30%, 10 to 30%, 20 to 30%, 8 to 20%, 8 to 10%, 8.3%, or 30%, but is not limited thereto.

In the present invention, the organic solvent may be one or more organic solvents selected from the group consisting of tetrahydrofuran (THF), ethyl acetate (EA), chloroform (CHCl₃), and dichloromethane (DCM), preferably an anhydrous organic solvent, and may be tetrahydrofuran (THF) or ethyl acetate (EA), but is not limited thereto.

The boiling points, evaporation degrees, and water solubilities of the tetrahydrofuran (THF), ethyl acetate (EA), chloroform (CHCl₃), and dichloromethane (DCM) are as shown in the following Table 1:

**[Table 1]**

| | Tetrahydrofuran (THF) | Ethyl acetate (EA) | Dichloromethane (DCM) | Chloroform (CHCl₃) |
|---|---|---|---|---|
| Boiling point (°C) | 66 | 77.1 | 39.6 | 61.2 |
| Evaporation degree (Torr, 20°C) | 142 | 73 | 350 | 158.4 |
| Water solubility (g/100 mL, 20°C) | 30 | 8.3 | 1.6 | 0.8 |

As used herein, the term "FP-CIT linear precursor" refers to a compound represented by the following Chemical Formula 1 as a starting material used in the synthesis of a radiopharmaceutical [¹⁸F]FP-CIT: 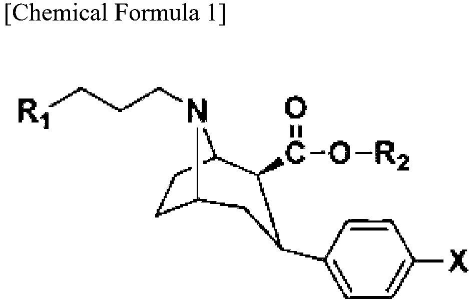 (in Chemical Formula 1,
R₁ is sulfonyl or a halogen,
R₂ is a C₁₋₅ alkyl, and
X is a halogen).

In the present invention, R₁ is methanesulfonyl (mesyl, 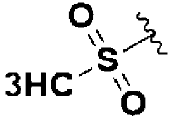 ), toluenesulfonyl (tosyl, 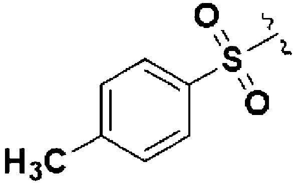 ), nitrobenzenesulfonyl (nosyl, 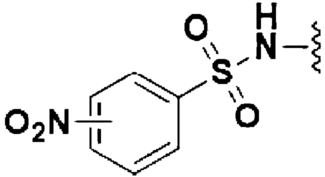 ), F, Cl, Br, or I,
R₂ is methyl ( 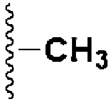 ) or ethyl ( 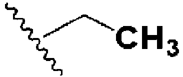 ), and
X may be Cl, Br, or I, and according to an exemplary embodiment of the present invention, the FP-CIT linear precursor may be N-(3-methansulfonyloxipropyl)-2-β-carbomethoxy-3-β-(4-iodophenyl)tropane or N-(3-toluenesulfonyloxipropyl)-2-β-carbomethoxy-3-β-(4-iodophenyl)tropane, but is not limited thereto.

In the present invention, the FP-CIT linear precursor, preferably the composition according to the present invention including the FP-CIT linear precursor may preferably be stored (preserved) at a temperature of -50 to 0°C, -40 to 0°C, -30 to 0°C, -20 to 0°C, -10 to 0°C, -50°C, -40°C, -30°C, -20°C, -10°C, or 0°C, and according to an exemplary embodiment of the present invention, the storage temperature of the FP-CIT linear precursor may be -20°C, but is not limited thereto.

In the present invention, the FP-CIT linear precursor may be stored at a concentration of 0.1 to 16 mg/mL, 0.1 to 15 mg/mL, 0.1 to 13 mg/mL, 0.1 to 10 mg/mL, 0.1 to 8 mg/mL, 0.1 to 5 mg/mL, 0.1 to 3 mg/mL, 0.1 to 2 mg/mL, 0.1 to 1 mg/mL, 0.5 to 16 mg/mL, 0.5 to 15 mg/mL, 0.5 to 13 mg/mL, 0.5 to 10 mg/mL, 0.5 to 8 mg/mL, 0.5 to 5 mg/mL, 0.5 to 3 mg/mL, 0.5 to 2 mg/mL, 0.5 to 1 mg/mL, 1 to 16 mg/mL, 1 to 15 mg/mL, 1 to 13 mg/mL, 1 to 10 mg/mL, 1 to 8 mg/mL, 1 to 5 mg/mL, 1 to 3 mg/mL, 1 to 2 mg/mL, 0.1 mg/mL, 0.5 mg/mL, 1 mg/mL, 2 mg/mL, 3 mg/mL, 4 mg/mL, 5 mg/mL, 6 mg/mL, 7 mg/mL, or 8 mg/mL, but the concentration is not limited thereto.

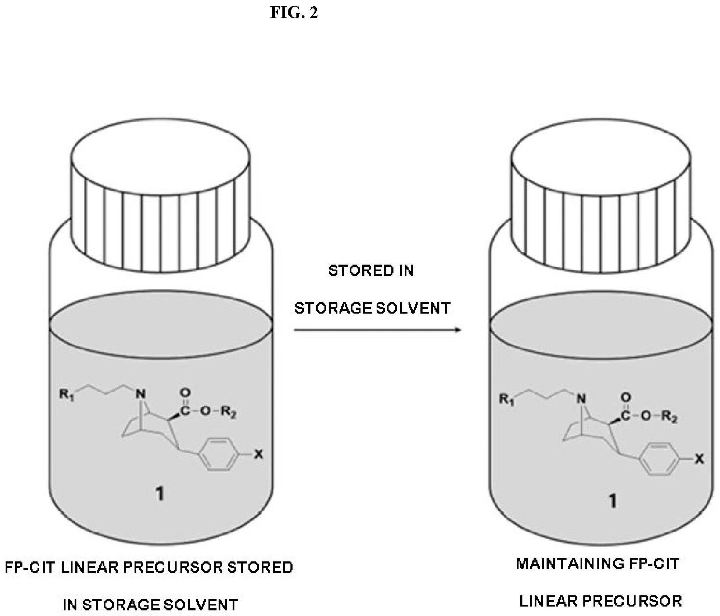

As used herein, "storage (preservation) of the FP-CIT linear precursor" refers to stabilizing an FP-CIT linear precursor having low chemical stability to suppress the conversion of the FP-CIT linear precursor into an FP-CIT cyclic salt precursor or the degradation of the FP-CIT linear precursor into various compounds, thereby allowing the FP-CIT linear precursor to maintain its linearity (see FIG. 2).

As used herein, "storage (preservation)" may refer to a state in which the FP-CIT linear precursor is dissolved in an organic solvent having the following characteristics:
(a) a boiling point of 10 to 100°C;
(b) an evaporation degree of 50 to 400 Torr; and
(c) a water solubility of 0.1 to 50%.

As used herein, the terms "storage of the FP-CIT linear precursor," "preservation of the FP-CIT linear precursor," "stabilization of the FP-CIT linear precursor," or "improvement in storage stability of the FP-CIT linear precursor" may be used interchangeably.

Therefore, the liquid composition for storing an FP-CIT linear precursor or the composition for improving the storage stability of an FP-CIT linear precursor of the present invention is characterized by being able to suppress the conversion of an FP-CIT linear precursor into an FP-CIT cyclic salt precursor and to stably store the FP-CIT linear precursor for a long period of time.

In addition, the improvement in storage stability of the FP-CIT linear precursor in the present invention may mean that the linearity of the FP-CIT linear precursor is maintained for a long period of time by suppressing the conversion of the FP-CIT linear precursor into an FP-CIT cyclic salt precursor to stabilize the FP-CIT linear precursor.

According to an exemplary embodiment of the present invention, the FP-CIT linear precursor may be maintained at 50 to 99%, 60 to 99%, 70 to 99%, 80 to 99%, 90 to 99%, 90 to 98%, 90 to 97%, 90 to 96%, 90%, 90.7%, 91%, 92%, 93%, 94%, 95%, 95.5%, or 96% of the initially stored amount of FP-CIT linear precursor when stored for 12 months, but the percentage is not limited thereto.

According to an exemplary embodiment of the present invention, when the FP-CIT linear precursor is stored for 12 months, 1 to 50%, 1 to 40%, 1 to 30%, 1 to 20%, 1 to 10%, 1 to 8%, 1 to 5%, 3 to 5%, 4 to 10%, 4.5 to 9.3%, 4%, 4.5%, 5%, 6%, 7%, 8%, 9.3%, or 10% of the initially stored amount of FP-CIT linear precursor may be converted to an FP-CIT cyclic salt precursor, but the percentage is not limited thereto.

According to an exemplary embodiment of the present invention, when the FP-CIT linear precursor is stored for 12 months, (FP-CIT cyclic salt precursor)/(FP-CIT linear precursor) may be 0.01 to 1, 0.01 to 0.8, 0.01 to 0.5, 0.01 to 0.3, 0.01 to 0.2, 0.01 to 0.1, 0.05 to 1, 0.05 to 0.8, 0.05 to 0.5, 0.05 to 0.3, 0.05 to 0.2, or 0.05 to 0.1, but is not limited thereto.

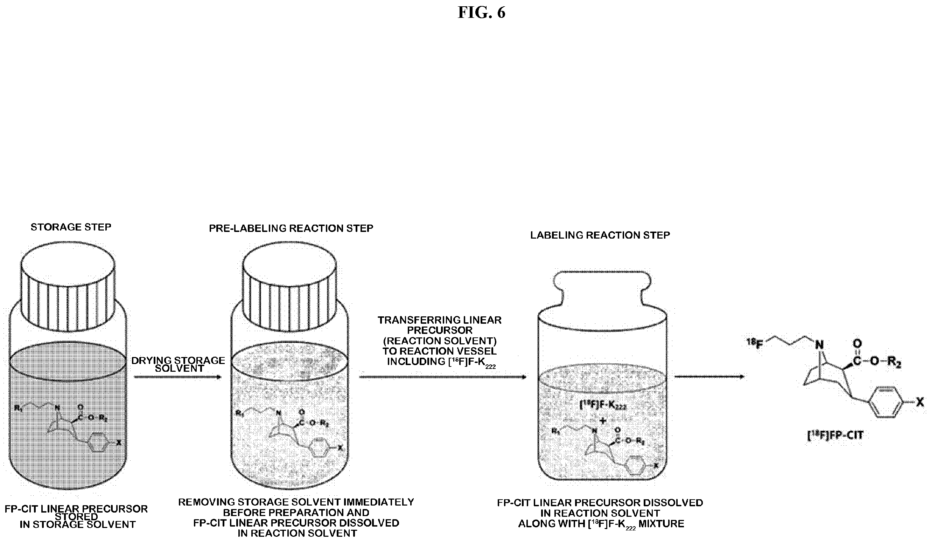

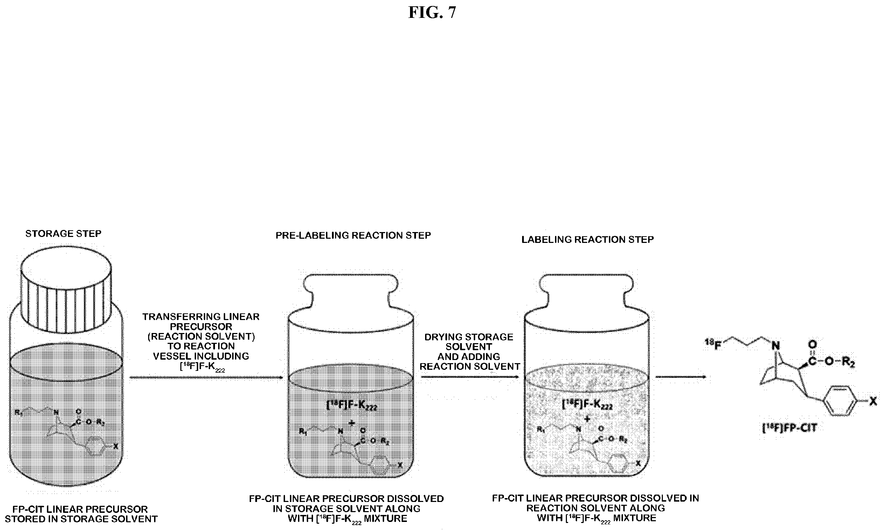

Furthermore, the present invention provides a method for preparing [¹⁸F]FP-CIT using the composition according to the present invention including an FP-CIT linear precursor as a starting material, the method including (see FIGS. 6 and 7):
(a) a step of isolating the FP-CIT linear precursor by removing an organic solvent from the composition according to the present invention including the FP-CIT linear precursor;
(b) a pre-labeling reaction step of preparing a mixture by dissolving the FP-CIT linear precursor isolated in Step (a) in a reaction solvent; and
(c) a labeling reaction step of labeling (with [¹⁸F]fluoride) by adding the mixture in Step (b) to a reaction vessel including activated [¹⁸F]fluoride.

Furthermore, the present invention provides a method for preparing [¹⁸F]FP-CIT using the composition according to the present invention including an FP-CIT linear precursor as a starting material, the method including (see FIGS. 6 and 7):
(a) a step of preparing a mixture by adding the composition according to the present invention including an FP-CIT linear precursor to a reaction vessel including activated [¹⁸F]fluoride;
(b) a pre-labeling reaction step of removing an organic solvent from the mixture in Step (a); and
(c) a labeling reaction step of labeling (with [¹⁸F]fluoride) by adding a reaction solvent to the mixture from which the organic solvent has been removed.

In the present invention, the [¹⁸F]FP-CIT is characterized by being prepared through a nucleophilic fluorination reaction of an FP-CIT linear precursor with activated [¹⁸F]fluoride.

In the present invention, the reaction solvent is a polar organic solvent, and the polar organic solvent may be one or more protic tertiary alcohols selected from the group consisting of t-butanol, t-amyl alcohol, and 1-methoxy-2-methyl-2-propanol; or
one or more aprotic organic solvents selected from the group consisting of acetonitrile (CH₃CN), dimethyl sulfoxide (DMSO), and dimethylformamide (DMF), but is not limited thereto.

As used herein, "activated [¹⁸F] fluoride" refers to an anhydrous [¹⁸F] fluoride ion which is generated in a cyclotron, then eluted with an anion exchange column and an eluent including a phase transition catalyst, and the like and dried, and is suitable for isotope labeling, and according to an exemplary embodiment of the present invention, the activated [¹⁸F]fluoride may be activated with tetra-n-butylammonium or 4,7,13,16,21,24-hexaoxa-1,10-diazabicyclo[8.8.8]hexacosane (Kryptofix^{™} 222), which is a phase transition catalyst, but the phase transition catalyst is not limited thereto.

In addition, the present invention provides a method for preparing [¹⁸F]FP-CIT, the method including: a step of pre-treating an FP-CIT linear precursor by dissolving the FP-CIT linear precursor in an organic solvent having the following characteristics:
(a) a boiling point of 10 to 100°C;
(b) an evaporation degree of 50 to 400 Torr; and
(c) a water solubility of 0.1 to 50%.

In the present invention, the method may further include the following steps, but is not limited thereto:
(a) a step of isolating an FP-CIT linear precursor by removing an organic solvent from the organic solvent in which the FP-CIT linear precursor is dissolved;
(b) a pre-labeling reaction step of preparing a mixture by dissolving the FP-CIT linear precursor isolated in Step (a) in a reaction solvent; and
(c) a labeling reaction step of labeling by adding the mixture in Step (b) to a reaction vessel including activated [¹⁸F]fluoride.

In the present invention, the method may further include the following steps, but is not limited thereto:
(a) preparing a mixture by adding an organic solvent in which an FP-CIT linear precursor is dissolved to a reaction vessel including activated [¹⁸F]fluoride;
(b) a pre-labeling reaction step of removing an organic solvent from the mixture in Step (a); and
(c) a labeling reaction step of labeling by adding a reaction solvent to the mixture from which the organic solvent has been removed.

Furthermore, the present invention provides a kit for storing an FP-CIT linear precursor, including the composition according to the present invention.

In the present invention, the kit may additionally include instructions in which a specific description (storage temperature, storage concentration, storage container, and the like) regarding the composition according to the present invention is recorded.

Further, the present invention provides a kit for preparing [¹⁸F]FP-CIT, including a first kit, a second kit, and instructions,
the first kit includes the composition according to the present invention, including the FP-CIT linear precursor, and
the second kit may include activated [¹⁸F]fluoride and a reaction solvent, but is not limited thereto.

In the present invention, the instructions may be instructions in which a specific description regarding the method for preparing [¹⁸F]FP-CIT according to the present invention is recorded.

### [Mode for Invention]

Hereinafter, preferred examples for helping with understanding of the present invention will be suggested. However, the following examples are provided only so that the present invention may be more easily understood, and the content of the present invention is not limited by the following examples.

### [Examples]

### Example 1. Confirmation of purity of FP-CIT linear precursor accordingto storage solvent

### 1-1. Tetrahydrofuran (THF)

N-(3'-(methansulfonyloxipropyl)-2-β-carbomethoxy-3-β-(4'-iodophenyl)tropane, a high-purity FP-CIT linear precursor, was stored at room temperature in tetrahydrofuran (THF), a storage solvent, at a concentration of 1 mg/mL, and a purity was confirmed for 12 months using HPLC analysis. The HPLC analysis was carried out under the following conditions: column (Luna C18 2504.6 mm), mobile phase (A:B = 0.1% trifluoroacetic acid:acetonitrile), analytical program (0 min; 100:0, 0 to 15 min; 30:70, 15 to 16 min; 100:0, 16 to 30 min; 100:0), flow rate (1 mL/min), and UV wavelength (254 nm).

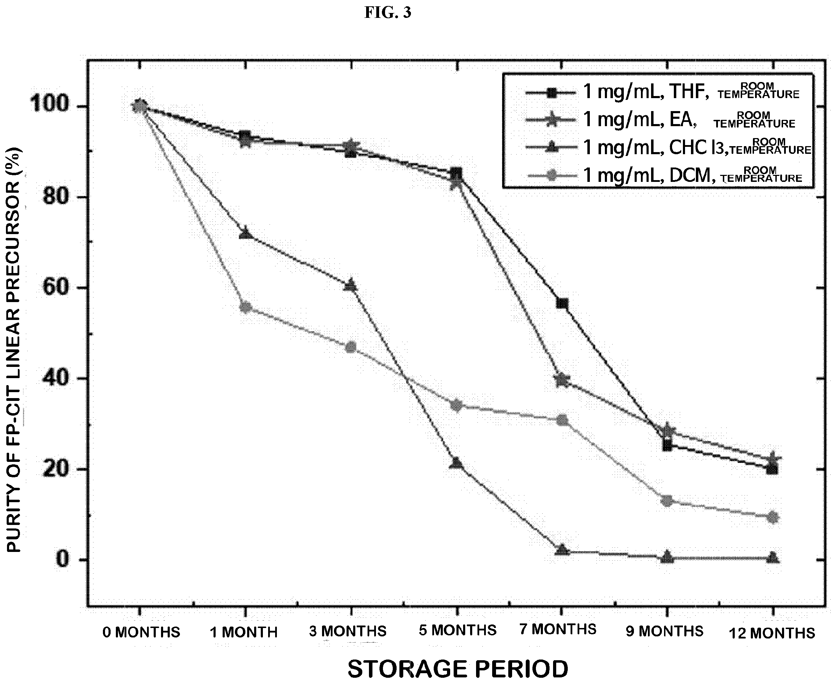

The results of the HPLC analysis showed that, when the purity at the start of storage was assumed to be 100%, the purity was maintained at 90% or more after one month of storage and 85% or more after five months of storage, but gradually decreased, and a purity of about 20% was observed after 12 months. The purity of the FP-CIT linear precursor over time is summarized in FIG. 3 and the following Table 2.

### 1-2. Ethyl acetate (EA)

HPLC analysis was carried out using the same experimental method and analytic conditions as in 1-1, except that the storage solvent was ethyl acetate (EA). The results of the HPLC analysis showed that, when the purity at the start of storage was assumed to be 100%, the purity was maintained at 90% or more after one month of storage and 80% or more after five months of storage, but gradually decreased, and a purity of about 22% was observed after 12 months. The purity of the FP-CIT linear precursor over time is summarized in FIG. 3 and the following Table 2.

### 1-3. Chloroform (CHCl₃)

HPLC analysis was carried out using the same experimental method and analytic conditions as in 1-1, except that the storage solvent was chloroform (CHCl₃). The results of the HPLC analysis showed that, when the purity at the start of storage was assumed to be 100%, the purity was maintained at 70% or more after one month of storage, but gradually decreased, and a purity of about 22% and a purity of about 1% or less were observed after 5 months and after 12 months, respectively. The purity of the FP-CIT linear precursor over time is summarized in FIG. 3 and the following Table 2.

### 1-4. Dichloromethane (DCM)

HPLC analysis was carried out using the same experimental method and analytic conditions as in 1-1, except that the storage solvent was dichloromethane (DCM). The results of the HPLC analysis showed that, when the purity at the start of storage was assumed to be 100%, the purity was maintained at 50% or more after one month of storage, but gradually decreased, and a purity of 34% and a purity of about 9% were observed after 5 months and after 12 months, respectively. The purity of the FP-CIT linear precursor over time is summarized in FIG. 3 and the following Table 2.

**[Table 2]**

| | **0 months** | **1 month** | **3 months** | **5 months** | **7 months** | **9 months** | **12 months** |
|---|---|---|---|---|---|---|---|
| **THF** | 100.0% | 93.4% | 89.9% | 85.3% | 56.6% | 25.6% | 20.2% |
| **EA** | 100.0% | 92.3% | 91.2% | 83.3% | 39.8% | 28.4% | 22.2% |
| **CHCl₃** | 100.0% | 71.8% | 60.4% | 21.2% | 2.2% | 0.7% | 0.5% |
| **DCM** | 100.0% | 55.8% | 47.0% | 34.2% | 30.9% | 13.1% | 9.5% |

### Example 2. Confirmation of purity of FP-CIT linear precursor according to storage temperature

### 2-1. Tetrahydrofuran (THF)

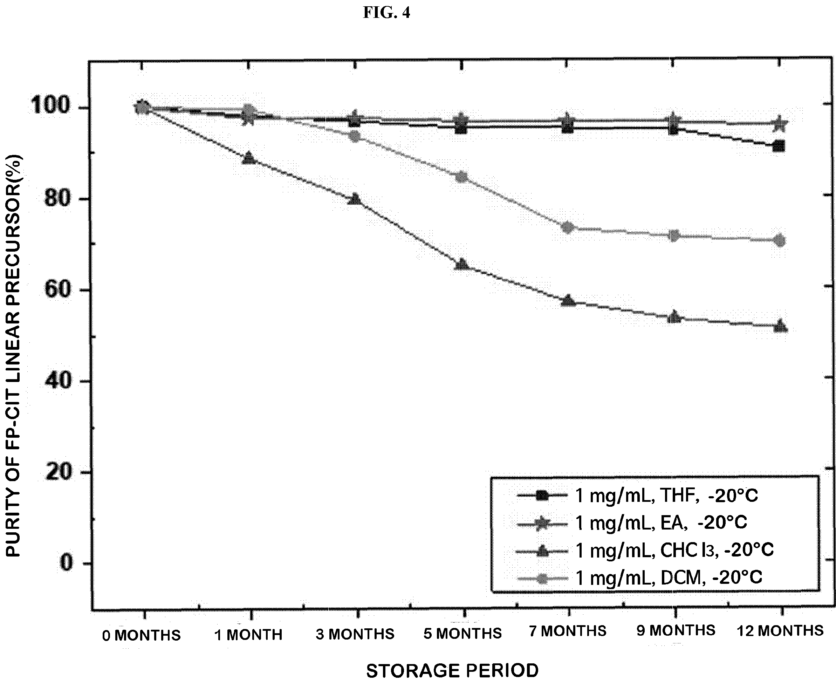

HPLC analysis was carried out using the same experimental method and analytic conditions as in Example 1-1, except that the storage temperature was -20°C. The results of the HPLC analysis showed that, when the purity at the start of storage was assumed to be 100%, the purity was maintained at 95% or more after five months of storage and at 90% or more even after 12 months of storage. The purity of the FP-CIT linear precursor over time is summarized in FIG. 4 and the following Table 3.

### 2-2. Ethyl acetate (EA)

HPLC analysis was carried out using the same experimental method and analytic conditions as in Example 1-2, except that the storage temperature was -20°C. The results of the HPLC analysis showed that, when the purity at the start of storage was assumed to be 100%, the purity was maintained at 95% or more even after twelve months of storage. The purity of the FP-CIT linear precursor over time is summarized in FIG. 4 and the following Table 3.

### 2-3. Chloroform (CHCl₃)

HPLC analysis was carried out using the same experimental method and analytic conditions as in Example 1-3, except that the storage temperature was -20°C. The results of the HPLC analysis showed that, when the purity at the start of storage was assumed to be 100%, the purity was maintained at 85% or more after one month of storage, but gradually decreased, and a purity of 65% and a purity of about 51% were observed after 5 months and after 12 months, respectively. The purity of the FP-CIT linear precursor over time is summarized in FIG. 4 and the following Table 3.

### 2-4. Dichloromethane (DCM)

HPLC analysis was carried out using the same experimental method and analytic conditions as in Example 1-4, except that the storage temperature was -20°C. The results of the HPLC analysis showed that, when the purity at the start of storage was assumed to be 100%, the purity was maintained at 95% or more after one month of storage and 80% or more after five months of storage, but gradually decreased, and a purity of about 70% was observed after 12 months. The purity of the FP-CIT linear precursor over time is summarized in FIG. 4 and the following Table 3.

**[Table 3]**

| | **0 months** | **1 month** | **3 months** | **5 months** | **7 months** | **9 months** | **12 months** |
|---|---|---|---|---|---|---|---|
| **THF** | 100.0% | 98.0% | 96.7% | 95.2% | 95.1% | 94.7% | 90.7% |
| **EA** | 100.0% | 97.5% | 97.4% | 96.7% | 96.4% | 96.4% | 95.5% |
| **CHCl₃** | 100.0% | 88.6% | 79.4% | 65.1% | 57.1% | 53.2% | 51.3% |
| **DCM** | 100.0% | 99.4% | 93.5% | 84.4% | 73.2% | 71.3% | 70.1% |

### Example 3. Confirmation of purity of FP-CIT linear precursor according to storage solvent, concentration thereof, and storage temperature

### 3-1. Tetrahydrofuran (THF) and room temperature

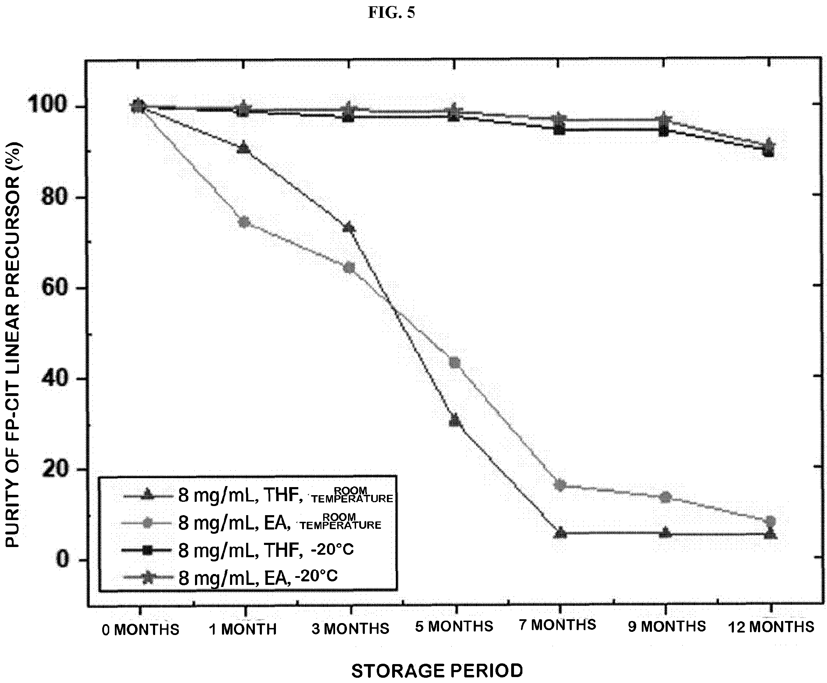

HPLC analysis was carried out using the same experimental method and analytic conditions as in Example 1-1, except that the storage concentration was 8 mg/mL. The results of the HPLC analysis showed that, when the purity at the start of storage was assumed to be 100%, the purity was maintained at 90% or more after one month of storage, but gradually decreased, and a purity of 30% and a purity of about 5% were observed after 5 months and after 12 months, respectively. The purity of the FP-CIT linear precursor over time is summarized in FIG. 5 and the following Table 4.

### 3-2. Ethyl acetate (EA) and room temperature

HPLC analysis was carried out using the same experimental method and analytic conditions as in Example 1-2, except that the storage concentration was 8 mg/mL. The results of the HPLC analysis showed that, when the purity at the start of storage was assumed to be 100%, the purity was maintained at 70% or more after one month of storage, but gradually decreased, and a purity of 43% and a purity of about 8% were observed after 5 months and after 12 months, respectively. The purity of the FP-CIT linear precursor over time is summarized in FIG. 5 and the following Table 4.

### 3-3. Tetrahydrofuran (THF) and - 20°C

HPLC analysis was carried out using the same experimental method and analytic conditions as in Example 3-1, except that the storage temperature was -20°C. The results of the HPLC analysis showed that, when the purity at the start of storage was assumed to be 100%, the purity was maintained at 95% or more after five months of storage and at 89% even after 12 months of storage. The purity of the FP-CIT linear precursor over time is summarized in FIG. 5 and the following Table 4.

### 3-4. Ethyl acetate (EA) and -20 °C

HPLC analysis was carried out using the same experimental method and analytic conditions as in Example 3-2, except that the storage temperature was -20°C. The results of the HPLC analysis showed that, when the purity at the start of storage was assumed to be 100%, the purity was maintained at 98% or more after five months of storage and at 90% or more even after 12 months of storage. The purity of the FP-CIT linear precursor over time is summarized in FIG. 5 and the following Table 4.

**[Table 4]**

| | **0 months** | **1 month** | **3 months** | **5 months** | **7 months** | **9 months** | **12 months** |
|---|---|---|---|---|---|---|---|
| **THF, room temperature** | 100.0% | 90.6% | 72.9% | 30.4% | 5.5% | 5.5% | 5.1% |
| **EA, room temperature** | 100.0% | 74.5% | 64.3% | 43.3% | 16.2% | 13.5% | 8.0% |
| **THF, -20°C** | 100.0% | 98.8% | 97.5% | 97.5% | 94.7% | 94.4% | 89.7% |
| **EA, -20°C** | 100.0% | 99.3% | 99.0% | 98.6% | 96.7% | 96.4% | 90.6% |

### Example 4. Synthesis of [¹⁸F]FP-CIT using FP-CIT linear precursor

### 4-1. Treatment of t-amyl alcohol as reaction solvent during storage solvent treatment process

[¹⁸F]FP-CIT was synthesized using 8 mg of FP-CIT linear precursor stored in EA and t-amyl alcohol as a reaction solvent (FIG. 6). A specific storage solvent treatment method and synthesis conditions were as follows:
A storage solvent was dried by heating the FP-CIT linear precursor stored in EA at 50°C or higher while blowing nitrogen gas into the FP-CIT linear precursor, and an FP-CIT linear precursor dissolved in the reaction solvent was prepared by adding a reaction solvent t-amyl alcohol thereto.

[¹⁸F]FP-CIT was synthesized by adding the FP-CIT linear precursor dissolved in t-amyl alcohol (dissolved in the reaction solvent) to a reaction vessel including an activated [¹⁸F]fluoride mixture ([¹⁸F]F-K₂₂₂) and heating the resulting mixture at 50°C or higher.

The reaction mixture was diluted with water, and unreacted [¹⁸F]fluoride and [¹⁸F]FP-CIT were separated using a C18 Sep-Pak to confirm the synthesis yield of [¹⁸F]FP-CIT. The synthesis yield of [¹⁸F]FP-CIT was 35%.

### 4-2. Treatment of t-amyl alcohol as reaction solvent during synthesis process of [¹⁸F]FP-CIT

[¹⁸F]FP-CIT was synthesized using 8 mg of FP-CIT linear precursor stored in EA and t-amyl alcohol as a reaction solvent (FIG. 7). A specific storage solvent treatment method and synthesis conditions were as follows:
A storage solvent was dried by adding 8 mg of FP-CIT linear precursor stored in EA to a reaction vessel including an activated [¹⁸F]fluoride mixture ([¹⁸F]F-K₂₂₂) and heating the resulting mixture at 50°C or higher while blowing nitrogen gas into the reaction vessel.

[¹⁸F]FP-CIT was synthesized by adding the reaction solvent t-amyl alcohol to a reaction vessel including activated [¹⁸F]fluoride and FP-CIT linear precursor, and then heating the resulting mixture at 50°C or higher. After the reaction mixture was purified, the synthesis yield of [¹⁸F]FP-CIT was 35.4%.

### 4-3. Treatment of acetonitrile as reaction solvent during synthesis process of [¹⁸F]FP-CIT

[¹⁸F]FP-CIT was synthesized using 8 mg of FP-CIT linear precursor stored in EA and acetonitrile as a reaction solvent, and a storage solvent treatment method and synthesis conditions were the same as in Example 4-2, except for the reaction solvent. As a result, after the reaction mixture was purified, the synthesis yield of [¹⁸F]FP-CIT was 17.2%.

The above-described description of the present invention is provided for illustrative purposes, and those skilled in the art to which the present invention pertains will understand that the present invention can be easily modified into other specific forms without changing the technical spirit or essential features of the present invention. Therefore, it should be understood that the above-described embodiments are only exemplary in all aspects and are not restrictive.

### [Industrial Applicability]

When an organic solvent having the characteristics according to the present invention is used for storing (preserving) an FP-CIT linear precursor, it is possible not only to maintain the FP-CIT linear precursor at high purity by suppressing conversion of the FP-CIT linear precursor into a cyclic salt form or degradation into various compounds, but also to synthesize a radiopharmaceutical [¹⁸F]FP-CIT at a high yield while using existing [¹⁸F]FP-CIT labeling conditions as they are, and thus the present invention has industrial applicability because it may be usefully used for the preparation of [¹⁸F]FP-CIT.

## Claims

1. A liquid composition for storing an FP-CIT linear precursor, comprising an organic solvent having the following characteristics:
(a) a boiling point of 10 to 100°C;
(b) an evaporation degree of 50 to 400 Torr; and
(c) a water solubility of 0.1 to 50%.

2. The liquid composition of claim 1, wherein the organic solvent is one or more selected from the group consisting of tetrahydrofuran (THF), ethyl acetate (EA), chloroform (CHCl₃), and dichloromethane (DCM).

3. The liquid composition of claims 1 or 2, wherein the FP-CIT linear precursor is a compound represented by the following Chemical Formula 1: 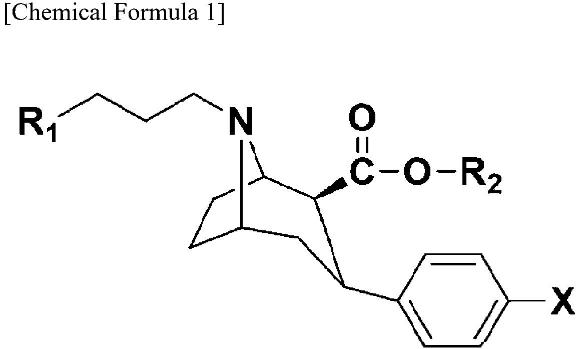 (in Chemical Formula 1,
R₁ is sulfonyl or a halogen,
R₂ is a C₁₋₅ alkyl, and
X is a halogen).

4. The liquid composition of claim 3, wherein R₁ is methanesulfonyl (mesyl), toluenesulfonyl (tosyl), nitrobenzenesulfonyl (nosyl), F, Cl, Br, or I,
R₂ is methyl or ethyl, and
X is Cl, Br, or I.

5. The liquid composition of any one of claims 1 to 4, wherein a storage temperature of the FP-CIT linear precursor is -50 to 0°C.

6. The liquid composition of any one of claims 1 to 5, wherein the FP-CIT linear precursor is stored at a concentration of 0.1 to 16 mg/mL.

7. The liquid composition of any one of claims 1 to 6, wherein the liquid composition suppresses the conversion of the FP-CIT linear precursor into an FP-CIT cyclic salt precursor.

8. The liquid composition of any one of claims 1 to 7, wherein the FP-CIT linear precursor maintains 50 to 99% of the initially stored amount of FP-CIT linear precursor when stored for 12 months.

9. The liquid composition of any one of claims 1 to 8, wherein, when the FP-CIT linear precursor is stored for 12 months, 1% to 50% of the initially stored amount of FP-CIT linear precursor is converted into an FP-CIT cyclic salt precursor.

10. The liquid composition of any one of claims 1 to 9, wherein the FP-CIT linear precursor has a ratio (FP-CIT cyclic salt precursor)/(FP-CIT linear precursor) of 0.01 to 1 when stored for 12 months.

11. A composition for improving the storage stability of an FP-CIT linear precursor, comprising an organic solvent having the following characteristics:
(a) a boiling point of 10 to 100°C;
(b) an evaporation degree of 50 to 400 Torr; and
(c) a water solubility of 0.1 to 50%.

12. The composition of claim 11, wherein the organic solvent is one or more selected from the group consisting of tetrahydrofuran (THF), ethyl acetate (EA), chloroform (CHCl₃), and dichloromethane (DCM).

13. The composition of claims 11 or 12, wherein the FP-CIT linear precursor is a compound represented by the following Chemical Formula 1: 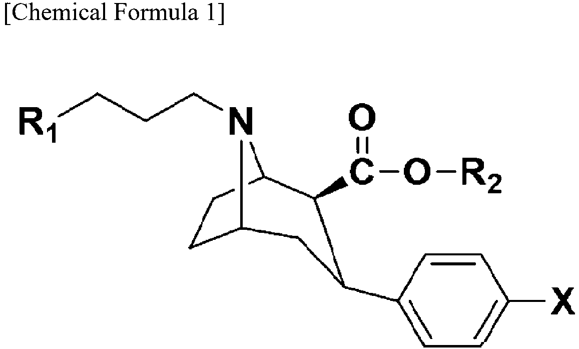 (in Chemical Formula 1,
R₁ is sulfonyl or a halogen,
R₂ is a C₁₋₅ alkyl, and
X is a halogen).

14. The composition of claim 13, wherein R₁ is methanesulfonyl (mesyl), toluenesulfonyl (tosyl), nitrobenzenesulfonyl (nosyl), F, Cl, Br, or I,
R₂ is methyl or ethyl, and
X is Cl, Br, or I.

15. The composition of any one of claims 11 to 14, wherein a storage temperature of the FP-CIT linear precursor is -50 to 0°C.

16. The composition of any one of claims 11 to 15, wherein the FP-CIT linear precursor is stored at a concentration of 0.1 to 16 mg/mL.

17. The composition of any one of claims 11 to 16, wherein the improvement in the storage stability of the FP-CIT linear precursor suppresses the conversion of the FP-CIT linear precursor into an FP-CIT cyclic salt precursor.

18. The composition of any one of claims 11 to 17, wherein the FP-CIT linear precursor maintains 50 to 99% of the initially stored amount of FP-CIT linear precursor when stored for 12 months.

19. The composition of any one of claims 11 to 18, wherein, when the FP-CIT linear precursor is stored for 12 months, 1% to 50% of the initially stored amount of FP-CIT linear precursor is converted into an FP-CIT cyclic salt precursor.

20. The composition of any one of claims 11 to 19, wherein the FP-CIT linear precursor has a ratio (FP-CIT cyclic salt precursor)/(FP-CIT linear precursor) of 0.01 to 1 when stored for 12 months.

21. A method for stabilizing an FP-CIT linear precursor by storing the FP-CIT linear precursor in the composition of any one of claims 1 to 10.

22. A method for preparing [¹⁸F]FP-CIT using the composition of any one of claims 1 to 10 comprising an FP-CIT linear precursor as a starting material, the method comprising:
(a) a step of isolating the FP-CIT linear precursor by removing an organic solvent from the composition of any one of claims 1 to 10 comprising the FP-CIT linear precursor;
(b) a pre-labeling reaction step of preparing a mixture by dissolving the FP-CIT linear precursor isolated in Step (a) in a reaction solvent; and
(c) a labeling reaction step of labeling by adding the mixture in Step (b) to a reaction vessel including activated [¹⁸F]fluoride.

23. A method for preparing [¹⁸F]FP-CIT using the composition of any one of claims 1 to 10 comprising an FP-CIT linear precursor as a starting material, the method comprising:
(a) a step of preparing a mixture by adding the composition of any one of claims 1 to 10 comprising an FP-CIT linear precursor to a reaction vessel comprising activated [¹⁸F]fluoride;
(b) a pre-labeling reaction step of removing an organic solvent from the mixture in Step (a); and
(c) a labeling reaction step of labeling by adding a reaction solvent to the mixture from which the organic solvent has been removed.

24. The method of claims 22 or 23, wherein the [¹⁸F]FP-CIT is prepared through a nucleophilic fluorination reaction of an FP-CIT linear precursor with activated [¹⁸F]fluoride.

25. The method of any one of claims 22 to 24, wherein the reaction solvent is a polar organic solvent.

26. The method of claim 25, wherein the polar organic solvent is one or more protic tertiary alcohols selected from the group consisting of t-butanol, t-amyl alcohol, and 1-methoxy-2-methyl-2-propanol.

27. The method of claims 25 or 26, wherein the polar organic solvent is one or more aprotic organic solvents selected from the group consisting of acetonitrile (CH₃CN), dimethyl sulfoxide (DMSO), and dimethylformamide (DMF).

28. The method of any one of claims 22 to 27, wherein the activated [¹⁸F]fluoride is activated with tetra-n-butylammonium or 4,7,13,16,21,24-hexaoxa-1,10-diazabicyclo[8.8.8]hexacosane (Kryptofix^{™} 222).

29. A method for preparing [¹⁸F]FP-CIT, the method comprising: an FP-CIT linear precursor pretreatment step of dissolving an FP-CIT linear precursor in an organic solvent having the following characteristics:
(a) a boiling point of 10 to 100°C;
(b) an evaporation degree of 50 to 400 Torr; and
(c) a water solubility of 0.1 to 50%.

30. The method of claim 29, further comprising the following steps:
(a) a step of isolating an FP-CIT linear precursor by removing an organic solvent from the organic solvent in which the FP-CIT linear precursor is dissolved;
(b) a pre-labeling reaction step of preparing a mixture by dissolving the FP-CIT linear precursor isolated in Step (a) in a reaction solvent; and
(c) a labeling reaction step of labeling by adding the mixture in Step (b) to a reaction vessel including activated [¹⁸F]fluoride.

31. The method of claims 29 or 30, further comprising the following steps:
(a) preparing a mixture by adding an organic solvent in which an FP-CIT linear precursor is dissolved to a reaction vessel comprising activated [¹⁸F]fluoride;
(b) a pre-labeling reaction step of removing an organic solvent from the mixture in Step (a); and
(c) a labeling reaction step of labeling by adding a reaction solvent to the mixture from which the organic solvent has been removed.

32. A kit for storing an FP-CIT linear precursor, comprising the liquid composition for storing the FP-CIT linear precursor of any one of claims 1 to 10.

33. A kit for preparing [¹⁸F]FP-CIT, comprising a first kit, a second kit, and instructions,
wherein the first kit comprises a liquid composition for storing the FP-CIT linear precursor of any one of claims 1 to 10, comprising an FP-CIT linear precursor, and
the second kit comprises activated [¹⁸F]fluoride and a reaction solvent.
